(19) Europäisches Patentamt
European Patent Office
Office européen des brevets

(11) Veröffentlichungsnummer: **0 131 864**
**B1**

(12) **EUROPÄISCHE PATENTSCHRIFT**

(45) Veröffentlichungstag der Patentschrift: **26.09.90**

(21) Anmeldenummer: **84107889.2**

(22) Anmeldetag: **06.07.84**

(51) Int. Cl.$^5$: **A 61 K 37/02,** C 07 K 3/12, C 09 K 15/06 // C12N9/96, A61K39/395, A61K35/16, A61K37/66

(54) **Gegen Denaturierung beständige, wässrige Proteinlösungen, Verfahren zu ihrer Herstellung und ihre Verwendung.**

(30) Priorität: **13.07.83 DE 3325223**

(43) Veröffentlichungstag der Anmeldung:
**23.01.85 Patentblatt 85/04**

(45) Bekanntmachung des Hinweises auf die Patenterteilung:
**26.09.90 Patentblatt 90/39**

(84) Benannte Vertragsstaaten:
**AT BE CH DE FR GB IT LI LU NL SE**

(56) Entgegenhaltungen:
**EP-A-0 018 609**
**WO-A-83/00288**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Thurow, Horst, Dr.**
**Parkstrasse 20**
**D-6233 Kelkheim (Taunus) (DE)**

**EP 0 131 864 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft wäßrige Lösungen von Proteinen mit einem Molekulargewicht oberhalb 8500 Dalton, die gegen Adsorption an Grenzflächen, gegen Denaturierung und gegen Präzipitation des Proteins geschützt sind, sowie Verfahren zur Herstellung solcher Lösungen. Die Erfindung betrifft ferner die Verwendung solcher stabilisierten Lösungen für therapeutische Zwecke vorzugsweise in Dosiergeräten zur Applikation von Arzneimitteln.

Es ist bekannt, daß gelöste Proteine an hydrophoben Grenzflächen (dazu gehört auch die Grenzfläche wäßrige Lösung/Luft) adsorbiert werden (C. W. N. Cumper und A. E. Alexander, Irans. Faraday Soc. *46*, 235 (1950)). Proteine sind amphiphile Substanzen, d.h. sie haben sowohl hydrophile als auch hydrophobe Bereiche. Die hydrophoben Bereiche bilden den Kontakt zur hydrophoben Grenzfläche.

Als Folge der Adsorption der Proteine an Grenzflächen werden verschiedene Sekundärreaktionen beobachtet. Es kann beispielsweise zur "Denaturierung", d.h. zu einer Formänderung der adsorbierten Proteinmoleküle (Änderung der Tertiär- und/oder Sekundärstruktur) kommen. Daneben kann Aggregation von adsorbierten Proteinmolekülen zu löslichen oder unlöslichen polymeren Formen erfolgen. So ist von vielen Proteinen eine Oberflächen-Aggregation bekannt, die sich z.B. als Trübung der Lösung oder als biologische Inaktivierung der Proteine beim Rühren oder Schütteln der wäßrigen Lösungen bemerkbar macht (A. F. Henson, I. R. Mitchell, P. R. Mussellwhite, J. Colloid Interface Sci. *32*, 162 (1970)). Diese Oberflächenadsorption und -aggregation ist besonders nachteilig in Apparaten zum Transport von Proteinlösungen z.B. in automatischen Dosiergeräten für Arzneimittel. In einigen Fällen kommt es auch zu chemischen Reaktionen der adsorbierten Proteine mit gelösten Substanzen (F. MacRitchie, J. Macromol. Sci., Chem., 4, 1169 (1970)).

Die beschriebenen Grenzflächenprozesse können wieterhin einem Protein immunogene Eigenschaften (d.h. die Fähigkeit, immunologische Abwehrreaktionen in einem Organismus zu induzieren) verleihen oder bereits vorhandene immunogene Eigenschaften verstärken. Außerdem können biologische Eigenschaften, wie enzymatische, serologische oder hormonelle Aktivitäten, verändert oder zerstört werden.

Eine spezielle Form der hydrophoben Grenzflächen bildet sich beim Gefrieren von wäßrigen Lösungen, z.B. bei der Gefriertrocknung von Proteinen. An diesen Grenzflächen kommt es ebenfalls zu den beschriebenen Denaturierungen von Proteinen (U.B. Hansson, Acta Chem. Scand., *22*, 483 (1968)).

Aus der EP—A1—18609 sind wäßrige Proteinlösungen bekannt, die zur Vermeidung der Denaturierung der darin befindlichen Proteine an Grenzflächen eine oberflächenaktive Substanz mit kettenförmiger Grundstruktur enthalten, deren Kettenglieder mindestens zur Hälfte methyl- oder ethylsubstituierte Oxyethylen-Einheiten sind. Ferner wird die Behandlung von Oberflächen mit solchen oberflächenaktiven Substanzen und deren Verwendung zur Handhabung und Reinigung von Proteinen beschrieben.

Weiterhin sind aus der WO—A1—83/002888 stabile wäßrige Insulinzubereitungen zur Verwendung in Insulindosiervorrichtungen bekannt, die Polyoxyethylene-($C_8$—$C_{15}$)-alkylether enthalten.

Es wurde überraschend gefunden, daß sich wäßrige Lösungen von Proteinen mit einem Molekulargewicht oberhalb 8500 Dalton besonders gut stabilisieren lassen durch das Zumischen von Substanzen, die durch die Formel I charakterisiert werden:

$$R^2O—X_n—R^3 \qquad (I)$$

Die Erfindung betrifft somit eine wäßrige Lösung eines Proteins mit einem Molekulargewicht oberhalb von 8500 Dalton, die gekennzeichnet ist durch einen Gehalt einer Verbindung der Formel I,

$$R^2O—X_n—R^3 \qquad (I)$$

in welcher $X_n$ eine Kette von n Gliedern der Formeln II oder III

—CH—CH$_2$—O—          —CH$_2$—CH—O—
  |                                    |
 $R^1$      (II)                  $R^1$      (III)

in beliebiger Reihenfolge ist, n 2 bis 200, vorzugsweise 4 bis 100, insbesondere 8 bis 50 und $R^1$ Wasserstoff, Methyl oder Ethyl bedeuten, wobei die Reste $R^1$ gleich oder verschieden sein können, jedoch in mehr als der Hälfte der Kettenglieder $R^1$=Wasserstoff vorkommt und $R^2$ und $R^3$ gleich oder verschieden sind und Wasserstoff oder einen organsichen Rest bedeuten. Bevorzugt sind solche Verbindungen der Formel I, in welcher einer der Reste $R^2$ oder $R^3$ für Wasserstoff steht. Falls $R^2$ bzw. $R^3$ für einen organischen Rest steht, so wird darunter vorzugsweise ein aliphatischer Rest mit 1 bis 20 C-Atomen, ein alicyclischer Rest mit 3 bis 10 C-Atomen, ein alicyclisch-aliphatischer Rest mit 4 bis 20 C-Atomen, eine aliphatische Estergruppe mit 2 bis 20 C-Atomen, ein aryl-aliphatischer Rest mit 7 bis 20 C-Atomen oder ein Arylrest mit 6—20 C-Atomen, insbesondere aber Akyl mit 1 bis 20 C-Atomen, Alkanoyl mit 2 bis 20 C-Atomen oder Alkylphenyl mit 1 bis 10 Alkyl-C-Atomen verstanden.

Beispiele für die Reste $R^2$ und $R^3$ sind Methyl, Ethyl, Propyl, Butyl, oder die Reste, die sich von

Laurylalkohol oder Myristalalkohol ableiten; Carboxalkylgruppen, die sich von der Eissgsäure, Propionsäure, Buttersäure, Palmitinsäure oder Stearinsäure ableiten und Nonylphenoxy.

Stabilisierende Zusätze dieser Art haben gegenüber denen aus der EP—A1—18609 bekannten Zusätzen den Vorteil, daß sie aufgrund der längeren Polyoxyethylen-Ketten ($R^1$=H) erhöhte Löslichkeit in wäßrigen Medien aufweisen. Zudem hat sich gezeigt, daß bei Zusatz von Stabilisatoren der beschriebenen Art größere Proteine, d.h. solche mit einem Molekulargewicht von mehr als 8500 Daltons, gegen Grenzflächenprozesse geschützt werden können.

Die erfindungsgemäßen Proteinlösungen können auch ein Gemisch mehrerer verschiedener Verbindungen der Formel I enthalten. Weiter können diesen Lösungen übliche Mittel zur Einstellung der Isotonie wie Glycerin, Natriumchlorid, Glucose oder ein ähnliches Kohlenhydrat, zur Konservierung wie Phenol, Kresol oder p-Hydroxybenzoesäuremethylester, zur Pufferung des pH-Wertes wie Natriumphosphat, Acetat, Citrat, Barbital oder Tris-(hydroxymethyl)-aminomethan und zur Erzielung einer Depot-Wirkung zugefügt werden.

Die Erfindung betrifft weiterhin ein Verfahren zur Herstellung dieser stabilen Proteinlösungen, das dadurch gekennzeichnet ist, daß man einer wäßrigen Proteinlösung eine oberflächenaktive Substanz der Formel I zusetzt.

Es wird angenommen, daß die erfindungsgemäßen oberflächenaktiven Substanzen in der Grenzfläche so geformt sind, daß die hydrophoben Bereiche der Blockpolymeren den Kontakt zur Grenzfläche bilden und daß die hydrophilen Polyoxyethylen-Bereiche in die wäßrige Phase ragen, so daß sie direkte Wechselwirkung zwischen gelöstem Protein und Grenzfläche verhindern.

Die relativ großen hydrophilen Polyoxyethylen-Bereiche der Stabilisatoren, die wahrscheinlich in die wäßrige Phase hineinragen, bewirken möglicherweise, daß nur eine grobmaschige Beladung der Grenzfläche erreicht wird, so daß kleinere Proteinmoleküle nicht an der Adsorption an die Grenzfläche gehindert werden können.

Dementsprechend eignen sich die erfindungsgemäßen Substanzen insbesondere zur Stabilisierung wäßriger Lösungen höhermoleckularer Proteine. Diese Proteine bestehen aus einer oder mehreren Polypeptidketten und können neben Aminosäuren noch andere Bausteine (Zucker, Lipide usw.) enthalten.

Die erfindungsgemäßen oberflächenaktiven Substanzen sind allgemein geeignet zur Stabilisierung solcher gelöster Proteine, deren Molekulargewicht oberhalb 8500 Dalton liegt und die an hydrophobe Grenzfläche adsorbieren können, wie z.B. Polypeptide, globuläre Proteine und zusammengesetzte Proteine (Proteide), insbesondere Glykoproteine.

Als Beispiele für solche Proteine seien genannt Proteohormone wie Proinsuline, Präpoinsuline, Enzyme, wie Neuraminidase, Galactosidase, Glycosyl-Transferasen, Asparaginase, Catalase, Streptokinase, Myoglobin, sowie Proteine mit anderen Funktionen wie Immunglobuline verschiedener Klassen und Spezies, Albumin, Blutgerinnungsfaktoren Interferone, Interleukine, Wachstums- und Differenzierungsfaktoren. Bevorzugt sind Proteine mit einem Molekulargewicht oberhalb etwa 30 000.

Die Erfindung betrifft ferner die Verwendung der oben definierten stabilen Proteinlösungen bei der Reinigung von Proteinen durch Kristallisation, Chromatographie oder Ultrafiltration sowie deren Verwendung für therapeutische Zwecke insbesondere in Dosiergeräten wie implantierten oder externen automatischen Pumpen.

Hydrophobe Oberflächen, die mit Proteinlösungen in Kontakt kommen, lassen sich vorteilhaft mit den oberflächenaktiven Substanzen der Formel I zur Vermeidung der Adsorption oder Denaturierung der Proteine vorbehandeln.

Die Herstellung der erfindungsgemäß zu verwendenden oberflächenaktiven Substanzen werden in an sich bekannter Weise durch kontrollierte Addition von Alkylenoxiden an Alkylendiglykole (oder an entsprechende Hydroxyverbindungen) hergestellt. Die endständige Hydroxylfunktionen können gegebenenfalls anschließend verestert oder veräthert werden. Eine allgemeine Vorschrift zur Herstellung eines geeigneten Blockpolymerisats ist in Beispiel 1a beschrieben.

Beispiel 1

a) In einem 30 1-Glaskolben mit Rührer, Heizbad, Rückflußkühler und einer Einrichtung zur Dosierung von Alkylenoxiden unter Stickstoff werden 152,1 g Propylenglykol und 125 g 49%ige Kalilauge vorgelegt. Durch Vakuumdestillation wird entwässert. Anschließend werden bei 120°C langsam unter Rühren nacheinander 4141 g Propylenoxid und 17170 g Ethylenoxid zugegeben. Nach beendeter Reaktion wird durch Zugabe von Milchsäure das Kaliumhydroxid neutralisiert. Durch Vakuumdestillation werden die leichtflüchtigen Anteile abgetrennt und das Produkt entwässert. Das mittlere Molekulargewicht des Produktes beträgt 8750 Dalton bei einem Gehalt an Polyoxyäthylen von 80 Gew.-% im Molekül.

b) 5 Proben mit jeweils 7 ml einer 0,1 %igen Lösung von Ei-Albumin in 0,01 M Phosphatpuffer, pH 7 und 5 gleiche Proben mit Zusatz eines Stabilisators von 0,1% (bezogen auf das Gewicht der Lösung) eines Blockpolymerisats bestehend aus einer linearen Kette von Polypropylenglykol mit einem mittleren Molekulargewicht von 1750 Dalton, der beidseitig jeweils 40% Polyethylenglykol anpolymerisiert waren, wurden in 10 ml Glasampullen eingeschmolzen. Die Testlösungen wurden auf einen Test-Tube-Rotator mit mit 20 cm Abstand von der Radachse montiert und im Brutschrank bei 37°C mit 60 UpM rotiert. Die Proben ohne Stabilisator zeigten nach 5 Tagen eine starke Trübung, hervorgerufen durch denaturiertes Protein. Im Gegensatz dazu waren die Proben, die den Stabilisator enthielten, nach mehreren Monaten noch klar.

Beispiel 2

Proben von jeweils 7 ml einer Lösung von 5% humaner Immunglobuline, einer Lösung von 0,5% Myoglobin (Pfed) und einer Lösung von 0,1% β-Galactosidase und gleiche Proben mit einem Zusatz von 0,1% (bezogen auf das Gewicht der Lösung) eines Blockpolymerisats bestehend aus einer linearen Kette von Polypropylenglykol mit einem mittleren Molekulargewicht von 1750 Dalton, der beidseitig jeweils 50% Polyethylenglykol anpolymerisiert waren, wurden in 10 ml Glasampullen eingeschmolzen.

Die Proben wurden wie im Beispiel 1 b beschrieben bei 37°C geschüttelt. Die Proben ohne Stabilisator waren nach wenigen Tagen trüb, im Falle der β-Galactosidase war die enzymatische Aktivität auf weniger als 3% des Anfangswertes abgefallen. Im Gegensatz dazu waren die Proben, die den Stabilisator enthielten, auch nach mehreren Wochen noch klar. Die enzymatische Aktivität war praktisch voll erhalten.

Beispiel 3

Es wurde eine Lösung hergestellt, die 1% humane Immunglobuline in 0,01 M Phosphatpuffer, pH 7, und zur Stabilisierung 0,2% (bezogen auf das Gewicht der Lösung) eines Blockcopolymerisats bestehend aus einer linearen Kette von Polypropylenglykol mit einem mittleren Molekulargewicht von 1750 Dalton, der beidseitig 40% Polyethylenglykol anpolymerisiert waren, enthielt.

Die Lösung wurde in ein automatisch gesteuertes Dosiergerät gefüllt. Auf einem Bewegungssimulator im Brutschrank bei 37°C förderte das Dosiergerät eine klare Lösung über mehrere Wochen. In der geförderten klaren Lösung wurden die Gehalte an den Immunglobulinen gemessen. Sie stimmten mit den Anfangswerten überein.

Der Versuch wurde mit einer 1%igen Immunglobulin-Lösung, die keine Stabilisator enthielt, wiederholt. Dabei entstanden Präzipitate in den Förderschläuchen des Dosiergerätes nach wenigen Tagen. Der klare Überstand enthielt nahezu keine Immunglobuline mehr.

Beispiel 4

5 Proben mit jeweils 7 ml einer Lösung von 350.000 Einheiten humanem Fibroblasten-Interferon in Phosphatpuffer, pH 7, und 5 analoge Proben, die zusätzlich 0,01% (bezogen auf das Gewicht der Lösung) folgender Verbindung

$$CH_3-(CH_2)_{12}-CH_2-O-(CH_2-CH_2-O-)_{12}-(CH_2-\underset{|}{\overset{CH_3}{CH}}-O-)_2-H$$

enthielt, wurden in 10 ml Glasampullen eingeschmolzen. Die Testlösungen wurden wie im Beispiel 1b beschrieben bei 37°C rotiert. In den 5 unstabilisierten Proben wurde nach 2 Tagen ein Verlust der biologischen Aktivität von mehr als 95% gemessen. Die biologische Aktivität des Interferons in den 5 Proben, die den Stabilisator enthielten, war auch nach mehreren Wochen unverändert.

**Patentansprüche für die benannten Vertragsstaaten: BE, CH, DE, FR, GB, IT, LI, LU, NL, SE**

1. Wäßrige Lösung eines Proteins mit einem Molekulargewicht oberhalb von 8500 Dalton, gekennzeichnet durch einen Gehalt einer Verbindung der Formel I,

$$R^2O-X_n-R^3 \qquad (I)$$

in welcher $X_n$ eine Kette von n Gliedern der Formeln II oder III

$$-\underset{R^1}{\underset{|}{CH}}-CH_2-O- \quad (II) \qquad -CH_2-\underset{R^1}{\underset{|}{CH}}-O- \quad (III)$$

in beliebiger Reihenfolge ist,

n 2 bis 200, vorzugsweise 4 bis 100 und

$R^1$ Wasserstoff, Methyl oder Ethyl bedeuten, wobei die Reste $R^1$ gleich oder verschieden sein können, jedoch in mehr als der Hälfte der Kettenglieder $R^1$=Wasserstoff vorkommt und

$R^2$ und $R^3$ gleich oder verschieden sind und Wasserstoff oder einen organischen Rest bedeuten.

2. Wäßrige Proteinlösung gemäß Anspruch 1, dadurch gekennzeichnet, daß $R^2$ und $R^3$ unabhängig voneinander Wasserstoff, einen aliphatischen Rest mit 1 bis 20 C-Atomen, einen alicyclischen Rest mit 3 bis 10 C-Atomen, einen alicyclisch-aliphatischen Rest mit 4 bis 20 C-Atomen, eine aliphatische Estergruppe mit 2 bis 20 C-Atomen, einen aryl-aliphatischen Rest mit 7 bis 20 C-Atomen oder einen Arylrest mit 6 bis 20 C-Atomen bedeuten.

3. Wäßrige Proteinlösung gemäß Anspruch 1 oder 2, dadurch gekennzeichnet, daß $R^2$ und $R^3$ unabhängig voneinander Wasserstoff, Alkyl mit 1 bis 20 C-Atomen, Alkanoyl mit 2 bis 20 C-Atomen oder Alkylphenyl mit 1 bis 10 Alkyl-C-Atomen bedeuten.

4. Wäßrige Proteinlösung gemäß einem der Ansprüche 1 bis 3, dadurch gekennzeichnet, daß die Lösung ein Gemisch von mindestens zwei verschiedenen Verbindungen der Formel I enthält.

5. Wäßrige Proteinlösung gemäß einem der Ansprüche 1 bis 4, dadurch gekennzeichnet, daß die Lösung übliche Zusätze zur Einstellung der Isotonie, zur Konservierung, zur Pufferung und/oder zur Erzielung einer Depot-Wirkung enthält.

6. Wäßrige Proteinlösung gemäß einem der Ansprüche 1 bis 5, dadurch gekennzeichnet, daß die Lösung mindestens zwei unterschiedliche Proteine enthält.

7. Verfahren zur Herstellung einer stabilen Proteinlösung gemäß einem der Ansprüche 1 bis 6, dadurch gekennzeichnet, daß man einer wäßrigen Proteinlösung eine oberflächenaktive Substanz gemäß einem der Ansprüche 1 bis 3 zusetzt.

8. Verfahren zur Behandlung von hydrophoben Oberflächen zur Beseitigung ihrer adsorbierenden und denaturierenden Wirkung auf Proteine, dadurch gekennzeichnet, daß man die Oberflächen mit einer wäßrigen Lösung einer oberflächenaktiven Substanz der im Anspruch 1 angegebenen allgemeinen Formel behandelt.

9. Verwendung von Lösungen nach einem der Ansprüche 1 bis 6 bei der Reinigung von Proteinen durch Kristillisation, Chromatographie oder Ultrafiltration.

10. Verwendung von Lösungen nach einem der Ansprüche 1 bis 6 zur Vermeidung der Adsorption von Proteinen an hydrophoben Oberflächen.

11. Wäßrige Proteinlösung gemäß einem der Ansprüche 1 bis 6 zur Verwendung für therapeutische Zwecke.

12. Wäßrige Proteinlösung gemäß einem der Ansprüche 1 bis 6 zur Verwendung in Dosiergeräten.

13. Verwendung von oberflächenaktiven Substanzen gemäß einem der Ansprüche 1 bis 3 zur Vorbehandlung von hydrophoben Oberflächen zur Vermeidung der Adsorption oder Denaturierung von Proteinen.

**Patentansprüche für den benannten Vertragsstaat: AT**

1. Verfahren zur Herstellung einer wäßrigen Lösung eines Proteins mit einem Molekulargewicht oberhalb von 8500 Dalton, enthaltend eine Verbindung der Formel I,

$$R^2O—X_n—R^3 \qquad (I)$$

in welcher $X_n$ eine Kette von n Gliedern der Formeln II oder III

$$\begin{array}{c}—CH—CH_2—O—\\ |\\ R^1\end{array} \quad (II) \qquad \begin{array}{c}—CH_2—CH—O—\\ |\\ R^1\end{array} \quad (III)$$

in beliebiger Reihenfolge ist,

n 2 bis 200, vorzugsweise 4 bis 100 und

$R^1$ Wasserstoff, Methyl oder Ethyl bedeuten, wobei die Reste $R^1$ gleich oder verschieden sein können, jedoch in mehr als der Hälfte der Kettenglieder $R^1$=Wasserstoff vorkommt und

$R^2$ und $R^3$ gleich oder verschieden sind und Wasserstoff oder einen organischen Rest bedeuten, dadurch gekennzeichnet, daß man einer wäßrigen Proteinlösung eine Oberflächenaktive Substanz der Formel I zusetzt.

2. Verfahren gemäß Anspruch 1, dadurch gekennzeichnet, daß in Formel I $R^2$ und $R^3$ unabhängig voneinander Wasserstoff, einen aliphatischen Rest mit 1 bis 20 C-Atomen, einen alicyclischen Rest mit 3 bis 10 C-Atomen, einen alicyclisch-aliphatischen Rest mit 4 bis 20 C-Atomen, eine aliphatische Estergruppe mit 2 bis 20 C-Atomen, einen aryl-aliphatischen Rest mit 7 bis 20 C-Atomen oder einen Arylrest mit 6 bis 20 C-Atomen bedeuten.

3. Verfahren gemäß Anspruch 1 oder 2, dadurch gekennzeichnet, daß in Formel I $R^2$ und $R^3$ unabhängig voneinander Wasserstoff, Alkyl mit 1 bis 20 C-Atomen, Alkanoyl mit 2 bis 20 C-Atomen oder Alkylphenyl mit 1 bis 10 Alkyl-C-Atomen bedeuten.

4. Verfahren gemäß einem der Ansprüche 1 bis 3, dadurch gekennzeichnet, daß man der Lösung ein Gemisch von mindestens zwei verschiedenen Verbindungen der Formel I zusetzt.

5. Verfahren gemäß einem der Ansprüche 1 bis 4, dadurch gekennzeichnet, daß man der Lösung übliche Zusätze zur Einstellung der Isotonie, zur Konservierung, zur Pufferung und/oder zur Erzielung einer Depot-Wirkung zusetzt.

6. Verfahren gemäß einem der Ansprüche 1 bis 5, dadurch gekennzeichnet, daß man eine Lösung mindestens zweier unterschiedlicher Proteine herstellt.

7. Verfahren zur Behandlung von hydrophoben Oberflächen zur Beseitigung ihrer adsorbierenden und denaturierenden Wirkung auf Proteine, dadurch gekennzeichnet, daß man die Oberflächen mit einer wäßrigen Lösung einer oberflächenaktiven Substanz der im Anspruch 1 angegebenen allgemeinen Formel behandelt.

8. Verwendung von Lösungen nach einem der Ansprüche 1 bis 6 bei der Reinigung von Proteinen durch Kristallisation, Chromatographie oder Ultrafiltration.

9. Verwendung von Lösungen nach einem der Ansprüche 1 bis 6 zur Vermeidung der Adsorption von Proteinen an hydrophoben Oberflächen.

10. Verfahren zur Herstellung einer wäßrigen Proteinlösung gemäß einem der Ansprüche 1 bis 6 zur Verwendung für therapeutische Zwecke.

11. Verfahren zur Herstellung einer wäßrigen Proteinlösung gemäß einem der Ansprüche 1 bis 6 zur Verwendung in Dosiergeräten.

12. Verwendung von oberflächenaktiven Substanzen gemäß einem der Ansprüche 1 bis 3 zur Vorbehandlung von hydrophoben Oberflächen zur Vermeidung der Adsorption oder Denaturierung von Proteinen.

**Revendications pour les Etats Contractants désignés: BE, CH, DE, FR, GB, IT, LI, LU, NL, SE**

1. Solution aqueuse d'une protéine de masse moléculaire supérieure à 8500 daltons, caractérisée en ce qu'elle contient un composé de formule I,

$$R^2O—X_n—R^3 \qquad (I)$$

dans laquelle $X_n$ représente une chaîne de n maillons de formules II ou III

$$—CH(R^1)—CH_2—O— \quad (II) \qquad —CH_2—CH(R^1)—O— \quad (III)$$

dans un ordre quelconque,

n vaut 2 à 200, de préférence 4 à 100 et

$R^1$ représente hydrogène, méthyle ou éthyle, les restes $R^1$ pouvant être identiques ou différents, mais $R^1$ est hydrogène dans plus de la moitié des maillons de la chaîne, et

$R^2$ et $R^3$ sont identiques ou différents et représentent l'hydrogène ou un reste organique.

2. Solution aqueuse de protéine selon la revendication 1, caractérisée en ce que $R^2$ et $R^3$ représentent, indépendamment l'un de l'autre, un atome d'hydrogène, un reste aliphatique comportant de 1 à 20 atomes de carbone, un reste alicyclique comportant de 3 à 10 atomes de carbone, un reste alicycloaliphatique comportant de 4 à 20 atomes de carbone, un groupe ester aliphatique comportant de 2 à 20 atomes de carbone, un reste arylaliphatique comportant de 7 à 20 atomes de carbone ou un reste aryle comportant de 6 à 20 atomes de carbone.

3. Solution aqueuse de protéine selon la revendication 1 ou 2, caractérisée en ce que $R^2$ et $R^3$ représent, indépendamment l'un de l'autre, un atome d'hydrogène, un alkyle comportant de 1 à 20 atomes de carbone, un alcanoyle comportant de 2 à 20 atomes de carbone ou un alkylphényle comportant de 1 à 10 atomes de carbone dans la partie alkyle.

4. Solution aqueuse de protéine selon l'une des revendications 1 à 3, caractérisée en ce que la solution renferme au moins deux composés différents de formule I.

5. Solution aqueuse de protéine selon l'une des revendications 1 à 4, caractérisée en ce que la solution renferme les additifs usuels pour ajuster l'isotonie, pour la conservation, pour tamponner et/ou pour obtenir une action retard.

6. Solution aqueuse de protéine selon l'une des revendications 1 à 5, caractérisée en ce que la solution renferme au moins deux protéines différentes.

7. Procédé de préparation d'une solution stable de protéine selon l'une des revendications 1 à 6, caractérisé en ce que l'on ajoute à une solution aqueuse de protéine une substance tensioactive selon l'une des revendications 1 à 3.

8. Procédé de traitement de surfaces hydrophobes pour éliminer leur action adsorbante et dénaturante sur les protéines, caractérisé en ce que l'on traite les surfaces avec une solution aqueuse d'une substance tensioactive ayant la formule générale indiquée dans la revendication 1.

9. Utilisation de solutions selon l'une des revendications 1 à 6, pour la purification des protéines par cristallisation, chromatographie ou ultrafiltration.

10. Utilisation de solutions selon l'une des revendications 1 à 6, pour éviter l'adsorption des protéines sur des surfaces hydrophobes.

11. Solution aqueuse de proteine selon l'une des revendications 1 à 6 pour son utilisation à des fins thérapeutiques.

12. Solution aqueuse de protéine selon l'une des revendications 1 à 6 pour son utilisation dans des doseurs.

13. Utilisation de substances tensioactives selon l'une des revendications 1 à 3 pour le prétraitement de surfaces hydrophobes afin d'éviter l'adsorption ou la dénaturation des protéines.

**Revendications pour l'Etat Contractant désigné: AT**

1. Procédé de préparation d'une solution aqueuse d'une protéine de masse moléculaire supérieure à 8500 daltons, contenant un composé de formule I,

$$R^2O—X_n—R^3 \qquad (I)$$

dans laquelle $X_n$ représente une chaîne de n maillons de formules II ou III

$$—CH(R^1)—CH_2—O— \quad (II) \qquad —CH_2—CH(R^1)—O— \quad (III)$$

dans un ordre quelconque,

n vaut 2 à 200, de préférence 4 à 100 et

$R^1$ représente hydrogène, méthyle ou éthyle,

les restes $R^1$ pouvant être identiques ou différents, mais $R^1$ est hydrogène dans plus de la moitié des maillons de la chaîne, et

$R^2$ et $R^3$ sont identiques ou différents et représentent l'hydrogène ou un reste organique, caractérisé en ce que l'on ajoute à une solution aqueuse de protéine une substance tensioactive de formule I.

2. Procédé selon la revendication 1, caractérisé en ce que, dans la formule I, $R^2$ et $R^3$ représentent, indépendamment l'un de l'autre, un atome d'hydrogène, un reste aliphatique comportant de 1 à 20 atomes de carbone, un reste alicyclique comportant de 3 à 10 atomes de carbone, un reste alicycloaliphatique comportant de 4 à 20 atomes de carbone, un groupe ester aliphatique comportant de 2 à 20 atomes de carbone, un reste arylaliphatique comportant de 7 à 20 atomes de carbone ou un reste aryle comportant de 6 à 20 atomes de carbone.

3. Procédé selon la revendication 1 ou 2, caractérisé en ce que, dans la formule I, $R^2$ et $R^3$ représentent, indépendamment l'un de l'autre, un atome d'hydrogène, un alkyle comportant de 1 à 20 atomes de carbone, un alcanoyle comportant de 2 à 20 atomes de carbone ou un alkylphényle comportant de 1 à 10 atomes de carbone dans la partie alkyle.

4. Procédé selon l'une des revendications 1 à 3, caractérisé en ce que l'on ajoute à la solution un mélange d'au moins deux composés différents de formule I.

5. Procédé selon l'une des revendications 1 à 4, caractérisé en ce que l'on ajoute à la solution les additifs usuels pour ajuster l'isotonie, pour la conservation, pour tamponner et/ou pour obtenir une action retard.

6. Procédé selon l'une des revendications 1 à 5, caractérisé en ce que l'on prépare une solution d'au moins deux protéines différentes.

7. Procédé de traitement de surfaces hydrophobes pour éliminer leur action adsorbante et dénaturante sur les protéines, caractérisé en ce que l'on traite les surfaces avec une solution aqueuse d'une substance tensioactive ayant la formule générale indiquée dans la revendication 1.

8. Utilisation de solutions selon l'une des revendications 1 à 6, pour la purification des protéines par cristallisation, chromatographie ou ultrafiltration.

9. Utilisation de solutions selon l'une des revendications 1 à 6, pour éviter l'adsorption des protéines sur des surfaces hydrophobes.

10. Procédé de préparation d'une solution aqueuse de protéine selon l'une des revendications 1 à 6 pour son utilisation à des fins thérapeutiques.

11. Procédé de préparation d'une solution aqueuse de protéine selon l'une des revendications 1 à 6 pour son utilisation dans les doseurs.

12. Utilisation de substances tensioactives selon l'une des revendications 1 à 3 pour le prétraitement de surfaces hydrophobes afin d'éviter l'adsorption ou la dénaturation des protéines.

**Claims for the designated Contracting States: BE, CH, DE, FR, GB, IT, LI, LU, NL, SE**

1. An aqueous solution of a protein with a molecular weight above 8,500 daltons, which contains a compound of the formula I

$$R^2O—X_n—R^3 \qquad (I)$$

in which $X_n$ is a chain of n members of the formula II or III

$$\underset{R^1}{—CH}—CH_2—O— \quad (II) \qquad —CH_2—\underset{R^1}{CH}—O— \quad (III)$$

in any desired sequence,

n denotes 2 to 200, preferably 4 to 100 and

$R^1$ denotes hydrogen, methyl or ethyl, it being possible for the radicals $R^1$ to be identical or different, but $R^1$ being hydrogen in more than half of the chain members, and

$R^2$ and $R^3$ are identical or different and denote hydrogen or an organic radical.

2. An aqueous protein solution as claimed in claim 1, in which $R^2$ and $R^3$ independently of one another denote hydrogen, an aliphatic radical with 1 to 20 carbon atoms, an alicyclic radical with 3 to 10 carbon atoms, an alicyclic-aliphatic radical with 4 to 20 carbon atoms, an aliphatic ester group with 2 to 20 carbon atoms, an aryl-aliphatic radical with 7 to 20 carbon atoms or an aryl radical with 6 to 20 carbon atoms.

3. An aqueous protein solution as claimed in either of claims 1 and 2, in which $R^2$ and $R^3$ independently of one another denote hydrogen, alkyl with 1 to 20 carbon atoms, alkanoyl with 2 to 20 carbon atoms or alkylphenyl with 1 to 10 alkyl-carbon atoms.

4. An aqueous protein solution as claimed in any one of claims 1 to 3, which contains a mixture of at least two different compounds of the formula I.

5. An aqueous protein solution as claimed in any one of claims 1 to 4, which contains customary additives for adjusting the isotonicity, for preservation, for buffering and/or for achieving a depot action.

6. An aqueous protein solution as claimed in any one of claims 1 to 5, which contains at least two different proteins.

7. A process for the preparation of a stable protein solution as claimed in any one of claims 1 to 6, which comprises adding a surface-active substance as claimed in any one of claims 1 to 3 to an aqueous protein solution.

8. A process for the treatment of hydrophobic surfaces to eliminate their adsorbent and denaturing effect on proteins, which comprises treating the surfaces with an aqueous solution of a surface-active substance of the formula given in claim 1.

9. The use of a solution as claimed in any one of claims 1 to 6 in the purification of proteins by crystallization, chromatography or ultrafiltration.

10. The use of a solution as claimed in any one of claims 1 to 6 for avoiding adsorption of proteins on hydrophobic surfaces.

11. An aqueous protein solution as claimed in any one of claims 1 to 6 for use for therapeutic purposes.

12. An aqueous protein solution as claimed in any one of claims 1 to 6 for use in metering apparatuses.

13. The use of a surface-active substance as claimed in any one of claims 1 to 3 for the pretreatment of hydrophobic surfaces to avoid adsorption or denaturing of proteins.

**Claims for the designated Contracting State: AT**

1. A process for the preparation of an aqueous solution of a protein with a molecular weight above 8,500 daltons, which contains a compound of the formula I

$$R^2O—X_n—R^3 \qquad (I)$$

in which $X_n$ is a chain of n members of the formula II or III

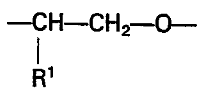 (II) 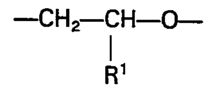 (III)

in any desired sequence,

n denotes 2 to 200, preferably 4 to 100 and

$R^1$ denotes hydrogen, methyl or ethyl, it being possible for the radicals $R^1$ to be identical or different, but $R^1$ being hydrogen in more than half of the chain members, and

$R^2$ and $R^3$ are identical or different and denote hydrogen or an organic radical, which comprises adding a surface-active substance of the formula I to an aqueous protein solution.

2. The process as claimed in claim 1, wherein, in formula I, $R^2$ and $R^3$ independently of one another denote hydrogen, an aliphatic radical with 1 to 20 carbon atoms, an alicyclic radical with 3 to 10 carbon atoms, an alicyclic-aliphatic radical with 4 to 20 carbon atoms, an aliphatic ester group with 2 to 20 carbon atoms, an aryl-aliphatic radical with 7 to 20 carbon atoms or an aryl radical with 6 to 20 carbon atoms.

3. The process as claimed in either of claims 1 and 2, wherein, in formula I, $R^2$ and $R^3$ independently of one another denote hydrogen, alkyl with 1 to 20 carbon atoms, alkanoyl with 2 to 20 carbon atoms or alkylphenyl with 1 to 10 alkyl-carbon atoms.

4. The process as claimed in any one of claims 1 to 3, wherein a mixture of at least two different compounds of the formula I is added to the solution.

5. The process as claimed in any one of claims 1 to 4, wherein customary additives for adjusting the isotonicity, for preservation, for buffering and/or for achieving a depot action are added to the solution.

6. The process as claimed in any of claims 1 to 5, wherein a solution which contains at least two different proteins is prepared.

7. A process for the treatment of hydrophobic surfaces to eliminate their adsorbent and denaturing

effect on proteins, which comprises treating the surfaces with an aqueous solution of a surface-active substance of the formula given in claim 1.

8. The use of a solution as claimed in any one of claims 1 to 6 in the purification of proteins by crystallization, chromatography or ultrafiltration.

9. The use of a solution as claimed in any one of claims 1 to 6 for avoiding adsorption of proteins on hydrophobic surfaces.

10. A process for the preparation of an aqueous protein solution as claimed in any one of claims 1 to 6 for use for therapeutic purposes.

11. A process for the preparation of an aqueous protein solution as claimed in any one of claims 1 to 6 for use in metering apparatuses.

12. The use of a surface-active substance as claimed in any one of claims 1 to 3 for the pretreatment of hydrophobic surfaces to avoid adsorption or denaturing of proteins.